# EUROPÄISCHE PATENTSCHRIFT

(11) **EP 1 289 927 B1**
(45) Veröffentlichungstag und Bekanntmachung des Hinweises auf die Patenterteilung: **14.05.2014**
(21) Anmeldenummer: 01945265.5
(22) Anmeldetag: 07.06.2001
(51) Int. Cl.: C07C 213/04

(54) **VERFAHREN ZUR HERSTELLUNG VON ETHANOLAMINEN**
METHOD FOR PRODUCING ETHANOLAMINES
PROCÉDÉ DE FABRICATION D'ÉTHANOLAMINES

(30) Priorität: 09.06.2000 DE 10028636
(43) Veröffentlichungstag der Anmeldung: 12.03.2003
(73) Patentinhaber: BASF SE, 67056 Ludwigshafen (DE)
(72) Erfinder: FRAUENKRON, Matthias, 67251 Freinsheim (DE); MÜLLER, Ulrich, 67435 Neustadt (DE); HARDER, Wolfgang, 69469 Weinheim (DE); UNGER, Jörg, 67459 Böhl-Iggelheim (DE); MELDER, Johann-Peter, 67459 Böhl-Iggelheim (DE); MEIER, Anton, 2950 Kapellen (BE); HIMMEL, Walter, 67269 Grünstadt (DE)
(86) Internationale Anmeldenummer: PCT/EP2001/006481
(87) Internationale Veröffentlichungsnummer: WO 2001/094290

(56) Entgegenhaltungen:
- WO-A-00/32553
- WO-A-96/11225
- WO-A-96/22274
- WO-A-99/33783

## Beschreibung

Die vorliegende Erfindung betrifft ein Verfahren zur Herstellung von Alkanolaminen aus Alkylenoxid und Ammoniak, wobei sich das erfindungsgemäße Verfahren dadurch auszeichnet, daß die Selektivität der Umsetzung durch die gezielte Steuerung der Temperatur des Reaktionsraums beeinflußt werden kann, wobei diese Steuerung durch Regelung des Temperaturprofils im Reaktionsraum erfolgt. In einer besonders bevorzugten Ausführungsform betrifft die Erfindung ein Verfahren zur flexiblen Reaktionsführung bei der Herstellung von Alkanolaminen, das sich dadurch auszeichnet, daß sich durch die vorgenannte Maßnahme der gezielten Temperaturregelung in einem Reaktionsraum in Anwesenheit des gleichen Katalysators die Produktselektivitäten der Alkanolaminsynthese einstellen lassen. In einer ebenfalls bevorzugten Ausführungsform betrifft die vorliegende Erfindung ein Verfahren zur Herstellung von Dialkanolaminen, bei dem in einer ersten Verfahrensstufe selektiv Monoalkanolamin und aus diesem Monoalkanolamin in einer zweiten Verfahrensstufe selektiv Dialkanolamin hergestellt wird.

DE-A 19 41 859.8 beschreibt ein Verfahren zur selektiven Synthese von Monoalkanolaminen aus Alkylenoxid und Ammoniak in Gegenwart eines Kationenaustauscherharzes. Zur optimalen Ausnutzung der Anlage werden dabei die Temperatur des Reaktionsvolumens des Katalysators und die Fließgeschwindigkeit der Reaktionsmischung durch den Katalysator so eingestellt, daß die höchsten Ausbeuten an Monoalkanolamin pro Zeiteinheit erzielt werden können.

Die EP-A 0 652 207 offenbart ein Verfahren zur Herstellung von Monoalkanolaminen aus Alkylenoxiden und Ammoniak in flüssiger Phase, wobei ein Katalysator verwendet wird, der ein Seltenerd-Element umfaßt, das auf einem hitzebeständigen Träger aufgebracht ist. Explizit wird beispielsweise die Herstellung von Monoethanolamin beschrieben. Die Temperatur, bei der die Reaktion erfolgt, wird dabei, wie sich aus den Beispielen ergibt, lediglich über die Temperatur des Ölbades bestimmt.

Die EP-A 0 941 986 beschreibt ein Verfahren zur Herstellung von Dialkanolaminen, ausgehend von Alkylenoxid und Ammoniak, wobei Zeolithkatalysatoren eingesetzt werden. Auch hier wird die Temperatur, bei der die Reaktion erfolgt, lediglich grob über die Temperatur des Ölbades bestimmt.

Die DD 298 636 beschreibt ein Verfahren zur Herstellung von Diethanolamin durch Umsetzung von Ammoniak und Ethenoxid in der Gasphase, wobei als Katalysator ein heterogener Katalysator, ein kristallines Silikat vom Pentasiltyp verwendet wird.

Die DE-A 25 47 328 beschreibt ein Verfahren zur kontinuierlichen Herstellung von Dialkanolaminen, in dem in einer ersten Reaktionszone ein Olefinoxid mit Ammoniak kontaktiert wird, das dabei gebildete Monoalkanolamin von dem aus der ersten Reaktionszone abziehenden Material abgetrennt wird und das abgetrennte Monoalkanolamin in einer zweiten Reaktionszone mit einem Olefinoxid kontaktiert wird. Die Einregelung der Trialkanolaminherstellung erfolgt dabei durch Einstellung des Molverhältnisses von Monoalkanolamin zu Olefinoxid, und die Umsetzungen finden mit Ausnahme von Wasser ohne Katalysator statt.

Eine Aufgabe der vorliegenden Erfindung war es, ein Verfahren bereitzustellen, das es ermöglicht, die Produktselektivität möglichst genau einzustellen und zu kontrollieren und damit ein Verfahren zur Hand zu haben, das unter anderem flexibel und schnell an einen bestimmten Produktbedarf anpaßbar ist und/oder hohe Produktselektivitäten ermöglicht.

Gelöst wird die Aufgabe durch ein Verfahren zur Herstellung von Ethanolaminen durch Umsetzung von Ammoniak mit Ethylenoxid in Gegenwart eines Katalysators in einem Reaktionsraum, bei dem
(i) in einer ersten Verfahrensstufe Monoethanolamin mit einer Selektivität von > 65 Gew.-%, bezogen auf die Gesamtmenge aus Mono-, Di- und Triethanolamin, hergestellt wird, und
(ii) in einer weiteren Verfahrensstufe Diethanolamin mit einer Selektivität von > 35% oder Triethanolamin mit einer Selektivität von > 35 Gew.-%, bezogen auf die Gesamtmenge aus Mono-, Di- und Triethanolamin, hergestellt wird,
   wobei die Verfahrensstufen (i) und (ii) im gleichen Reaktionsraum und in Gegenwart des gleichen Katalysators bei einem Druck von 20 bis 250 bar und einer Verweilzeit von 2 bis 60 Minuten durchgeführt werden,
   wobei
   die erste Verfahrensstufe (i) bei einer Maximaltemperatur im Reaktionsraum von 20 bis 180 °C, wobei die Temperaturdifferenz zwischen Maximaltemperatur und Temperatur der nicht vermischten Edukte 0 bis 100 °C beträgt, durchgeführt wird,
   und
   die weitere Verfahrensstufe (ii), sofern Diethanolamin mit einer Selektivität von > 35 Gew.-% hergestellt wird, bei einer Maximaltemperatur von 70 bis 200 °C, wobei die Temperaturdifferenz zwischen Maximaltemperatur und Temperatur der nicht vermischten Edukte 50 bis 120 °C beträgt, durchgeführt wird
   oder, sofern Triethanolamin mit einer Selektivität von > 35 Gew.-% hergestellt wird, bei einer Maximaltemperatur im Reaktionsraum von 75 bis 400 °C, wobei die Temperaturdifferenz zwischen Maximaltemperatur und Temperatur der nicht vermischten Edukte 70 bis 300 °C beträgt, durchgeführt wird,
   wobei die Verfahrensstufe (ii) auch vor der Verfahrensstufe (i) durchgeführt werden kann.

Die vorliegende Erfindung betrifft ein Verfahren zur Herstellung mindestens eines Alkanolamins durch Umsetzung von Ammoniak mit Alkylenoxid in einem Reaktionsraum in Gegenwart eines Katalysators unter Erhalt von Monoalkanolamin oder Dialkanolamin oder Trialkanolamin oder eines Gemischs aus zwei oder drei dieser Verbindungen, wobei die Verteilung der verschiedenen Alkanolamine im Produktspektrum über die Temperatur im Reaktionsraum gesteuert wird, das dadurch gekennzeichnet ist, daß die Temperatur durch Regelung des Temperaturprofils im Reaktionsraum eingestellt wird.

Der Begriff "Reaktionsraum" bezeichnet im Rahmen der vorliegenden Erfindung sowohl Reaktoren als auch Reaktorkompartimente als auch Reaktorabschnitte. Daher kann ein einzelner Reaktor sowohl einen einzigen Reaktionsraum darstellen als auch, wenn dieser einzelne Reaktor aus verfahrenstechnischen Gründen in zwei oder mehr Abschnitte, gegebenenfalls physikalisch voneinander getrennte Abschnitte oder Kompartimente, eingeteilt wird, aus zwei oder mehr verschiedenen Reaktionsräumen. Ebenso umfaßt der Begriff "Reaktionsraum" auch solche Ausführungsformen, in denen zwei oder mehr beispielsweise parallel oder seriell geschaltete Reaktoren einen einzigen Reaktionsraum darstellen.

Die Regelung des Temperaturprofils im Reaktionsraum ist nach sämtlichen geeigneten Methoden durchführbar. Beispielsweise kann von einem bestimmten Sollwert des Temperaturprofils ausgegangen werden, wobei der über geeignete Methoden bestimmte Istwert mit diesem Sollwert verglichen wird und über geeignete Methoden der Istwert diesem Sollwert iterativ oder unmittelbar, diskret oder kontinuierlich angepaßt wird. Ebenso ist es auch möglich, den Sollwert des Temperaturprofils unter Berücksichtigung beispielsweise des erhaltenen Produktspektrums zu ändern und den Istwert an die jeweiligen unterschiedlichen Sollwerte und damit an variable Sollwerte anzupassen. Mit dem Begriff "Sollwert" wird im Rahmen der vorliegenden Anmeldung derjenige Wert des Temperaturprofils bezeichnet, für den eine bestimmte erwünschte Verteilung der Alkanolamine im Produktspektrum erhalten wird. Mit dem Begriff "Istwert" wird im Rahmen der vorliegenden Anmeldung derjenige Wert des Temperaturprofils bezeichnet, der über geeignete Methoden ermittelt und dem Sollwert angepaßt wird. Mit dem Begriff "Wert des Temperaturprofils" wird im Rahmen der vorliegenden Anmeldung die Gesamtheit der über geeignete Meßmethoden bestimmten Meßwerte bezeichnet, die das Temperaturprofil im Reaktionsraum festlegen.

Die Regelung der Temperatur bzw. des Temperaturprofils erfolgt im wesentlichen durch Anpassung der Wärmeströme aus dem Reaktionsraum in die den Reaktionsraum umgebende Isolationseinrichtung, beispielsweise ein den Reaktionsraum umgebendes Doppelmantelkühlrohr. Bei dieser Ausführungsform werden, wie nachfolgend noch ausgeführt, weiter bevorzugt mehrer Doppelmantelkühlrohre nacheinander entlang des Reaktionsraums, beispielsweise eines Rohrreaktors, verwendet. Diese werden unabhängig voneinander mit Kühlflüssigkeit beschickt. Durch diese Anordnung ist die flexible Steuerung des Temperaturprofils erleichtert. Steuerungsgrößen sind bei vorgegebenem Reaktordesign und Reaktandenströmen mit festgelegten Zusammensetzungen die Volumenströme des Kühlmittels und die Eintrittstemperaturen in der Isolationseinrichtung, also beispielsweise den Doppelmantel bzw. in die aufeinander folgenden Doppelmäntel um den Reaktor.

Der variable Volumenstrom des Kühlmittels wird z.B. durch die Ansteuerung entsprechender Kreislaufpumpen realisiert. Die unterschiedliche Eintrittstemperatur des Kühlmittels in die Isolationseinrichtung kann durch einen oder mehreren Sekundärkreisläufe mit Wärmetauscher in weiten Grenzen variiert werden.

Das Temperaturprofil im Reaktionsraum kann generell über sämtliche geeigneten Methoden erfolgen. Insbesondere kann die Zahl und Lage der Meßpunkte, an denen die Temperatur bestimmt wird, an die Geometrie und Größe des Reaktionsraumes angepaßt werden. Diesbezüglich kann also das Temperaturprofil im Reaktionsraum mit beliebiger und den Anforderungen entsprechender Genauigkeit bestimmt werden.

Die Temperaturmessung an sich kann hierbei nach sämtlichen geeigneten Methoden durchgeführt werden.

Zur Temperaturmessung können im Innern des Reaktionsraumes alle gängigen Temperatursensoren eingesetzt werden, wobei beispielshaft Thermoelemente oder Widerstandthermometer, mit oder ohne Schutzrohr, verwendet werden können. Zur Temperaturmessung am Eintritt und Austritt des Kühlmediums in der Isolationseinrichtung werden vorzugsweise gleichartige Sensoren verwendet. Zusätzlich können an der äußeren Begrenzung der Isolationseinrichtung optische, berührungslose Methoden zur Messung der Wärmestrahlung für die Temperaturmessung benutzt werden oder die Messung erfolgt durch Thermoelemente bzw. Widerstandsthermometer mit Berührungsmessungen an der äußeren Begrenzung der Isolationseinrichtung. Bevorzugt erfolgt die Temperaturmessung im Innern des Reaktionsraumes in Thermoschutzrohren. Als Sensoren werden Prozeßleitsystem-bedingt entweder Thermoelemente oder Widerstandsthermometer eingesetzt.

Als besonders bevorzugte Meßgrößen zur Temperaturkontrolle sind im Rahmen des erfindungsgemäßen Verfahrens vor allem der Temperaturgradient und derjenige Ort im Reaktionsraum, an dem die Maximaltemperatur erreicht wird, zu nennen. Unter dem Begriff "Temperaturgradient" wird in diesem Zusammenhang die Temperaturdifferenz zwischen der Maximaltemperatur im Reaktor und der Temperatur des Eduktstromes oder der Eduktströme verstanden. Sollten demgemäß zwei oder mehr Eduktströme mit unterschiedlicher Temperatur in den Reaktionsraum geleitet werden, so ist es denkbar, daß ausgehend von einer Maximaltemperatur zwei oder mehr verschiedene Temperaturgradienten zur Temperaturkontrolle herangezogen werden. Ebenso ist es denkbar, daß, je nach Geometrie oder/und Größe des Reaktionsraumes, zwei oder mehr Orte im Reaktionsraum exisitieren, an denen jeweils die im Rahmen der Meßgenauigkeit oder/und der Genauigkeitsanforderungen der Verfahrensführung gleichen Maximaltemperaturen auftreten.

Für die Materialbeanspruchung des Reaktors ist es besonders vorteilhaft nicht eine örtlich scharf begrenzte besonders heisse Reaktionszone zu verwenden, sondern einen ausgeglichenen Temperaturgradienten über die Länge des Rohres. Zusätzlich ist die Wahl der Hotspot-Temperatur und der Ort des Hotspots längs des gewünschten Verhältnisses der Produkte vorteilhaft. Sie ergeben sich aus der Wahl der Volumenströme (Verweilzeiten) und dem somit eingestellten Temperaturniveau im Reaktor.

Daher betrifft die vorliegende Erfindung auch ein Verfahren, wie oben beschrieben, das dadurch gekennzeichnet ist, daß die Regelung des Temperaturprofils im Reaktionsraum über gezielte Festlegung des Temperaturgradienten im Reaktionsraum oder über gezielte Festlegung des Ortes der Maximaltemperatur im Reaktionsraum oder über gezielte Festlegung beider Parameter erfolgt.

Der Temperaturgradient beschreibt die örtliche Änderung der Temperatur längs der Reaktionsstrecke. Dazu werden mehrere Temperaturfühler im Reaktionsraum entlang dieser Strecke verteilt. Mit diesen kann die örtliche Änderung der Temperatur und ihr maximaler Wert, der Hotspot, ermittelt werden, wobei die Regelung wie hierin weiter oben beschrieben erfolgt.

Das Temperaturprofil, das im Rahmen des erfindungsgemäßen Verfahrens im Reaktionsraum auftritt und geregelt wird, kann durch sämtliche geeigneten Maßnahmen beeinflußt werden.

Zur Temperaturerniedrigung kann beispielsweise die im Laufe der Umsetzung erzeugte Wärme durch sämtliche geeigneten Methoden abgeführt werden. Unter anderem ist eine externe Kühlung, etwa durch einen oder mehrere Kühlmäntel, die den Reaktionsraum umgeben, denkbar. Weiter ist es möglich, freiwerdende Reaktionswärme durch Durchleiten eines oder mehrerer geeigneter Inertgase durch den Reaktionsraum abzuführen. Ebenso kann Reaktionswärme beispielsweise durch Verdampfen eines Teils des sich im Reaktionsraum befindlichen Ammoniaks entfernt werden. Zur Temperaturerhöhung im Reaktionsraum kann ebenfalls auf sämtliche geeigneten Methoden zurückgegriffen werden. Darunter fallen sowohl direkte Methoden wie beispielsweise das Erwärmen des Reaktionsraumes von außen als auch indirekte Methoden. Als indirekte Methoden werden unter anderem solche Maßnahmen verstanden, bei denen Temperaturerhöhung durch vollständiges oder teilweises Reduzieren der oben beschriebenen temperaturerniedrigenden Maßnahmen erreicht wird.

Sollte es im Rahmen des erfindungsgemäßen Verfahren zur Regelung des Temperaturprofils erforderlich sein, kann in Abhängigkeit von der Geometrie des Reaktionsraumes in einem oder mehreren Abschnitten die Temperatur erhöht und in einem oder mehreren Abschnitten gleichzeitig die Temperatur erniedrigt werden.

Weitere Möglichkeiten zur Beeinflussung der Temperatur im Reaktionsraum sind weiter die Verweilzeit der Reaktanden im Reaktionsraum, die Durchflußrate der Reaktionsmischung durch den Reaktionsraum bei kontinuierlicher Verfahrensführung oder auch die Temperatur des Eduktstromes oder der Eduktströme, die in den Reaktionsraum eingeleitet werden.

Vorzugsweise wird das Temperaturprofil durch die freigesetzte chemische Energie mit der korrespondierenden Aufheizung des Reaktandenstroms bestimmt und durch die physikalische Wärmeabfuhr aufgrund von Strahlung oder wirkungsvoller durch Wärmeabfuhr mittels eines Kühlmediums geregelt. Hierzu verweisen wir auch auf die obige detaillierte Beschreibung betrefend die bewußte Steuerung der Wärmeabfuhr unter Verwendung einer Kühleinrichtung, die es ermöglicht, die Wärmeabfuhr in weiten Grenzen zu steuern.

Unter die Gruppe der "Reaktanden", wie er obenstehend verwendet wird, fallen sämtliche Verbindungen, die im Laufe des erfindungsgemäßen Verfahrens miteinander reagieren. Vor allem sind dies Alkylenoxid, Ammoniak sowie Alkanolamin. Weitersind beispielshaft auch Monoalkanolamin, das mit Alkylenoxid zu Dialkanolamin reagieren kann, als auch Dialkanolamin, das mit Alkylenoxid zu Trialkanolamin reagieren kann, zu nennen.

Selbstverständlich können sämtliche dieser Maßnahmen auch in geeigneter Weise miteinander kombiniert werden. Als weitere Methode, die ebenfalls mit den vorgenannten Maßnahmen kombiniert werden kann, ist das Molverhältnis der Edukte Ammoniak und Alkylenoxid zu nennen. In dieser ganz besonders bevorzugten Ausführungsform des erfindungsgemäßen Verfahrens wird demgemäß die Verteilung der verschiedenen Alkanolamine im Produktspektrum durch das Molverhältnis der Edukte der Umsetzung gesteuert, die in den Reaktionsraum eingeleitet werden.

Daher betrifft die vorliegende Erfindung auch ein Verfahren, wie oben beschrieben, das dadurch gekennzeichnet ist, daß die Verteilung der verschiedenen Alkanolamine im Produktspektrum zusätzlich durch das Molverhältnis von Ammoniak und Alkylenoxid gesteuert wird.

Weiter ist es prinzipiell möglich, die Verteilung der verschiedenen Alkanolamine im Produktspektrum zusätzlich durch den Druck zu beeinflussen, unter dem die Reaktion durchgeführt wird.

Im allgemeinen exisitieren hinsichtlich der Geometrie und der Größe des Reaktionsraumes keine Beschränkungen. Insbesondere sind beispielsweise Rührkessel, Rührkesselkaskaden, Rohrreaktoren, Rohrreaktorkaskaden oder auch Reaktivdestillationskolonnen als geeignete Reaktionsräume einsetzbar. Auch sind sowohl Batchfahrweise als auch kontinuierliche Verfahrensführungen möglich. Kombinationen aus Batchfahrweise und kontinuierlicher Verfahrensführung sowie Kombinationen verschiedener Reaktorformen, die wiederum seriell oder/und parallel geschaltet sein können, sind ebenso denkbar.

In einer besonders bevorzugten Ausführungsform des erfindungsgemäßen Verfahrens werden zur Umsetzung von Alkylenoxid mit Ammoniak Rohrreaktoren eingesetzt. Diesbezüglich können zwei oder mehr Rohrreaktoren in Serie geschaltet sein oder zwei oder mehr Rohrreaktoren parallel geschaltet sein oder auch eine Kombination aus serieller und paralleler Schaltung vorgesehen sein.

Daher betrifft die vorliegende Erfindung auch ein Verfahren, wie oben beschrieben, das dadurch gekennzeichnet ist, daß die Umsetzung in einem Rohrreaktor durchgeführt wird.

Der erfindungsgemäß verwendete Rohreaktor ist beispielsweise ein gegenüber Druck beständiges Reaktionsrohr, das in einzelne Abschnitte logisch oder physikalisch aufteilbar ist. Die Abschnitte werden entweder gemeinsam oder einzeln unabhängig voneinander beispielsweise durch sie umgebende Kühlmittelkreisläufe, also durch z.B. Doppelmantelrohre oder andere den Wärmefluß bestimmende konstruktive Maßnahmen temperiert. In den einzelnen Abschnitten wird die Temperatur durch mehrere, d.h. mindestens zwei Temperatursensoren bestimmt.

Die konstruktive Anordnung der Abschnitte kann auch realisiert werden, indem mehrere konzentrisch ineinander angeordnete Reaktionsrohre nacheinander durch das Reaktionsgemisch derart durchströmt werden, daß die Wärmeabfuhr nicht nur auf das Kühlmedium in die umgebenden Doppelmantelrohre übertragen wird, sondern ebenfalls oder statt dessen zur Beheizung des Reaktionsgemisches selbst dient.

Die Umsetzung an sich wird im allgemeinen in Anwesenheit eines Katalysators durchgeführt.

Generell ist der Einsatz eines homogenen Katalysators, wie z.B. Wasser oder eines Alkanolamins denkbar. Unter anderem sind hier geeignete anorganische oder organische Säuren oder Ammoniumsalze zu nennen.

Besonders bevorzugt wird im Rahmen des erfindungsgemäßen Verfahrens ein heterogener Katalysator eingesetzt. Selbstverständlich können auch zwei oder mehr geeignete heterogene Katalysatoren verwendet werden. Ferner können zeolithanaloge Materialien, wie z.B. Alumo- und Silicoalumophosphate, sowie organische Ionenaustauscher, wie sie beispielsweise in der DE-A 19 41 859.8 beschrieben sind, eingesetzt werden.

In einer ganz besonders bevorzugten Ausführungsform wird als der mindestens eine heterogene Katalysator ein Zeolith-Katalysator eingesetzt. Mit dem Begriff "Zeolith-Katalysator" werden im Rahmen der vorliegenden Erfindungen sämtliche als Katalysatoren geeignete Oxide bezeichnet, die eine Zeolithstruktur oder eine zeolithanaloge Struktur aufweisen oder umfassen.

Daher betrifft die vorliegende Erfindung auch ein Verfahren, wie oben beschrieben, das dadurch gekennzeichnet ist, daß die Umsetzung in Gegenwart eines heterogenen Katalysators, bevorzugt eines heterogenen Zeolithkatalysators, durchgeführt wird.

Der erfindungsgemäß bevorzugt eingesetzte Katalysator ist vorzugsweise ein Oxid, umfassend mindestens die Elemente Si und Ti, mindestens nicht-kristallines Siliciumdioxid und mindestens eine kristalline Silikatphase, die mindestens eine Zeolithstruktur aufweist, wobei nicht-kristallines Siliciumdioxid auf mindestens einer kristallinen Silikatphase, die mindestens eine Zeolithstruktur aufweist, aufgebracht ist, dadurch gekennzeichnet, daß das Oxid keine Silicium-Kohlenstoff-Bindungen aufweist.

Zeolithe an sich sind bekanntermaßen kristalline Alumosilikate mit geordneten Kanal- und Käfigstrukturen, die Mikroporen aufweisen. Der Begriff "Mikroporen", wie er im Rahmen der vorliegenden Erfindung verwendet wird, entspricht der Definition in "Pure Appl. Chem." 57 (1985) S. 603-619, und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht über die bekannten Strukturen findet sich beispielsweise bei W. M. Meier, D. H. Olson und Ch. Baerlocher in "Atlas of Zeolite Structure Types", Elsevier, 4. Auflage, London 1996.

Im besonderen existieren Zeolithe, die kein Aluminium enthalten und bei denen im Silikatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Im einzelnen sind etwa die im erfindungsgemäßen Verfahren bevorzugt eingesetzten Katalysatoren Zeolithe mit Pentasil- Struktur, insbesondere solche mit den Strukturen ABW, ACO, AEI, AEL, AEN, AET, AFG, AFI, AFN, AFO, AFR, AFS, AFT, AFX, AFY, AHT, ANA, APC, APD, AST, ATN, ATO, ATS, ATT, ATV, AWO, AWW, BEA, BIK, BOG, BPH, BRE, CAN, CAS, CFI, CGF, CGS, CHA, CHI, CLO, CON, CZP, DAC, DDR, DFO, DFT, DOH, DON, EAB, EDI, EMT, EPI, ERI, ESV, EUO, FAU, FER, GIS, GME, GOO, HEU, IFR, ISV, ITE, JBW, KFI, LAU, LEV, LIO, LOS, LOV, LTA, LTL, LTN, MAZ, MEI, MEL, MEP, MER, MFI, MFS, MON, MOR, MSO, MTF, MTN, MTT, MTW, MWW, NAT, NES, NON, OFF, OSI, PAR, PAU, PHI, RHO, RON, RSN, RTE, RTH, RUT, SAO, SAT, SBE, SBS, SBT, SFF, SGT, SOD, STF, STI, STT, TER, THO, TON, TSC, VET, VFI, VNI, VSV, WEI, WEN, YUG, ZON oder einer Mischstruktur aus zwei oder mehr dieser Strukturen sowie ITQ-4, ITQ-6, ITQ-7 und CIT-6 zu nennen. Eine Vielzahl dieser Zeolithe dieses Typs sind beispielsweise in der oben genannten Literaturstelle von Meier et al. beschrieben.

Das erfindungsgemäß als Katalysator verwendete Oxid kann weiterhin titanhaltige Zeolithe mit der Struktur des UTD-1, CIT-1, CIT-5, MCM-22 oder MCM-61 umfassen. Als weitere titanhaltige Zeolithe sind solche mit der Struktur des ZSM-48 oder ZSM-12 zu nennen. Derartige Zeolithe sind unter anderem in der US-A 5 430 000 und der WO 94/29408 beschrieben, deren diesbezüglicher Inhalt vollumfänglich in die vorliegende Anmeldung durch Bezugnahme aufgenommen wird. Als besonders bevorzugt sind im Rahmen der vorliegenden Erfindung Ti-Zeolithe mit MFI-, MEL- oder MFI/MEL-Mischstruktur anzusehen. Als ebenfalls bevorzugt sind auch Ti-Zeolithe mit einer zu beta- Zeolith isomorphen Gerüststruktur zu nennen.

Außer Silicium und Titan kann die mindestens eine kristalline Silikatphase mit mindestens einer Zeolithstruktur auch zusätzliche Elemente wie z.B. Aluminium, Zirkon, Vanadium, Zinn, Zink, Eisen, Tellur, Niob, Tantal, Chrom, Kobalt, Nickel, Gallium, Germanium, Bor oder geringe Mengen an Fluor enthalten. Vorzugsweise umfaßt das Oxid Titan-, Vanadium-, Chrom-, Niob-, Zirkonium-Zeolithe, weiter bevorzugt Titan-Zeolithe und insbesondere Titansilicalite.
Was die Porenstruktur der mindestens einen kristallinen Silikatphase mit Zeolithstruktur anbelangt, so existieren diesbezüglich keine besonderen Beschränkungen. So sind Strukturen mit Mikroporen, mit Mesoporen oder mit Makroporen oder mit Mikro- und Mesoporen oder mit Mikro- und Makroporen oder mit Mikro- und Meso- und Makroporen denkbar, wobei die Definition dieser Poren im Rahmen der vorliegenden Erfindung der Definition in "Pure. Appl. Chem." 45, S. 71 ff. entspricht und Mikroporen mit einem Durchmesser von kleiner oder gleich 2 nm, Mesoporen mit einem Durchmesser von größer 2 nm bis ungefähr 50 nm und Makroporen mit einem Durchmesser von größer als 50 nm charakterisiert.

Was das Herstellungsverfahren des Oxides anbelangt, so exisitieren im wesentlichen keine Beschränkungen, solange aus diesem Verfahren das erfindungsgemäße Oxid erhalten wird. Bevorzugt wird das Oxid in einem Verfahren hergestellt, in dem ein geeignetes oxidisches Material, das mindestens eine kristalline Silikatphase mit Zeolithstruktur aufweist, mit einem geeigneten Silan oder Silanderivat behandelt wird.

Daher betrifft die vorliegende Erfindung auch ein Verfahren, wie oben beschrieben, das dadurch gekennzeichnet ist, daß der Katalysator ein silylierter Zeolithkatalysator ist.

Bevorzugt wird dieser Katalysator durch ein Verfahren zur Herstellung eines Oxids, umfassend mindestens die Elemente Si und Ti, mindestens nicht-kristallines Siliciumdioxid und mindestens eine kristalline Silikatphase, erhalten, in dem
(a) ein oxidisches Material, umfassend mindestens die Elemente Si und Ti und mindestens eine kristalline Silikatphase, die mindestens eine Zeolithstruktur aufweist, hergestellt wird und
(b) das aus (a) erhaltene oxidische Material
   (i) in mindestens einem Lösungsmittel umgesetzt wird mit mindestens einem Silan oder mindestens einem Silanderivat oder mit einem Gemisch aus zwei oder mehr davon unter Erhalt einer Mischung, umfassend mindestens ein oxidisches Umsetzungsprodukt und das mindestens eine Lösungsmittel,
      aus der Mischung direkt im Anschluß an die Umsetzung das mindestens eine Lösungsmittel unter Erhalt des mindestens einen oxidischen Umsetzungsproduktes entfernt wird und
      direkt im Anschluß an die Entfernung des mindestens einen Lösungsmittels das mindestens eine oxidische Umsetzungsprodukt unter Erhalt des Oxides calciniert wird, oder
   (ii) in der Gasphase umgesetzt wird mit mindestens einem Silan oder mindestens einem Silanderivat oder mit einem Gemisch aus zwei oder mehr davon unter Erhalt mindestens eines oxidischen Umsetzungsproduktes und direkt im Anschluß an die Umsetzung das mindestens eine oxidische Umsetzungsprodukt unter Erhalt des Oxides calciniert wird.

Details zu diesem Verfahren können der DE-A 199 54 322.4 entnommen werden, die diesbezüglich durch Bezugnahme in den Kontext der vorliegenden Anmeldung einbezogen wird.

In einer bevorzugten Ausführungsform wird das mindestens eine Silan oder das mindestens eine Silanderivat ausgewählt aus der Gruppe bestehend aus Trichlorsilan, Siliciumtetrachlorid, Methylhydrogendichlorsilan, Mono-, Di- und Trimethylchlorsilan, Tetraalkylorthosilikaten mit gleichen oder voneinander verschiedenen Alkylresten mit mehr als 2 C-Atomen, Hydrolysaten dieser Tetraalkylorthosilikate, Alkylalkoxysilanen mit gleichen oder voneinander verschiedenen Alkylresten und Alkoxyresten, und den vorgenannten Silanen oder Silanderivaten, die zusätzlich eine oder mehrere funktionelle Gruppen, ausgewählt aus der Gruppe bestehend aus Hydroxy-, Carboxyl-, Vinyl-, Glycidyl-, Amino-und Aminoalkylgruppen, aufweisen.

Besonders bevorzugt sind im Rahmen der vorliegenden Erfindung solche Silane oder Silanderivate, die mindestens eine Silicium-Kohlenstoff-Bindung aufweisen. Bevorzugt wird daher das mindestens eine Silan oder das mindestens eine Silanderivat ausgewählt aus der Gruppe bestehend aus Methylhydrogendichlorsilan, Mono-, Di- und Trimethylchlorsilan, Tetraalkylorthosilikaten mit gleichen oder voneinander verschiedenen Alkylresten mit mehr als 2 C-Atomen, Hydrolysaten dieser Tetraalkylorthosilikate, Alkylalkoxysilanen mit gleichen oder voneinander verschiedenen Alkylresten und Alkoxyresten, und den vorgenannten Silanen oder Silanderivaten, die zusätzlich eine oder mehrere funktionelle Gruppen, ausgewählt aus der Gruppe bestehend aus Hydroxy-, Carboxyl-, Vinyl-, Glycidyl-, Amino- und Aminoalkylgruppen, aufweisen.

Weiter ganz besonders bevorzugt wird ein oxidisches Material in Form eines Formkörpers, hergestellt aus Titansilikalit der TS-1-Struktur und Siliciumdioxidbinder, mit 3-Aminopropyltriethoxysilan, gelöst in einem geeigneten wasserfreien Lösungsmittel, umgesetzt.

Bevorzugt wird der Katalysator im Festbett verwendet. Als Einsatzformen sind auch gestaffelte oder strukturierte Packungen sowie Dünnschichtkatalysatoren zu nennen.

Dieser bevorzugt eingesetzte heterogene Zeolithkatalysator kann im Rahmen des erfindungsgemäßen Verfahrens generell gemäß sämtlicher geeigneter Methoden regeneriert werden. Solche Verfahren sind beispielsweise in der DE-A 100 15 246.5 beschrieben, die diesbezüglich durch Bezugnahme in den Kontext der vorliegenden Anmeldung einbezogen wird.

Als Alkylenoxide können im Rahmen des erfindungsgemäßen Verfahrens generell sämtliche geeigneten eingesetzt werden, wobei insbesondere solche mit der Struktur R₁R₂COCR₃R₄ bevorzugt sind. Dabei sind R₁ bis R₄ gleich oder verschieden voneinander und stehen für Wasserstoff, eine Methylgruppe oder eine Ethylgruppe. Besonders bevorzugt werden Alkylenoxide mit 2 bis 4 Kohlenstoffatomen eingesetzt, wobei wiederum bevorzugt Ethylenoxid verwendet wird.

Daher betrifft die vorliegende Erfindung auch ein Verfahren, wie oben beschrieben, das dadurch gekennzeichnet ist, daß als Alkylenoxid Ethylenoxid eingesetzt wird.

Hierbei kann das Alkylenoxid nach prinzipiell jedem geeigneten Verfahren hergestellt und in das erfindungsgemäße Verfahren eingesetzt werden. Derartige Verfahren sind Stand der Technik und werden u.a. in Ullmann's Enzyclopäde der Technischen Chemie (5. Auflage) ausführlich beschrieben. Weiterhin verweisen wir auf die Herstellungsverfahren von Alkylenoxiden und insbesondere von Propylenoxid, wie sie u.a. in der PCT/EP99/05740 und der DE-A 100 15 246.5 beschrieben sind, die diesbezüglich vollumfänglich durch Bezugnahme in den Kontext der vorliegenden Anmeldung einbezogen wird.

In einer bevorzugten Ausführungsform wird das erfindungsgemäß als Edukt verwendete Alkylenoxid durch Umsetzung des entsprechenden Alkens mit einem Hydroperoxid, einem Sauerstoff enthaltenden Gas oder reinem Sauerstoff hergestellt.

Daher betrifft die vorliegende Erfindung auch ein Verfahren, wie oben beschrieben, das dadurch gekennzeichnet ist, daß das Alkylenoxid hergestellt wird durch Umsetzung eines Alkens mit einem Hydroperoxid.

Wird als Edukt beispielsweise Propylenoxid eingesetzt, so wird dieses bevorzugt durch Umsetzung von Propen mit Wasserstoffperoxid hergestellt. Die Umsetzung mit Wasserstoffperoxid erfolgt weiter bevorzugt in Anwesenheit eines Katalysators, bevorzugt eines heterogenen Katalysators, weiter bevorzugt eines Katalysators, der eine Zeolithstruktur aufweist. Bezüglich der möglichen Zeolithstrukturen kann auf die obenstehend beschriebenen Strukturen verwiesen werden. Als besonders bevorzugt ist ein Katalysator mit der Struktur TS-1 zu nennen. Hierzu verweisen wir auf die oben bereits erwähnten Druckschriften PCT/EP99/05740 und DE-A 100 15 246.5.

Bezüglich der Herstellung des bevorzugt eingesetzten Ethylenoxids verweisen wir auf die oben zitierte Stelle in Ullmann's Encyklopädie der Technischen Chemie.

Sollte im erfindungsgemäßen Verfahren bevorzugt Monoalkanolamin hergestellt werden, so kann der Produktstrom aus dem Reaktionsraum, der neben dem Monoalkanolamin gegebenenfalls Dialkanolamin oder/und Trialkanolamin sowie Ammoniak und Wasser enthält, einer oder mehreren Trennstufen zugeführt werden, in denen dieses Gemisch aufgetrennt wird. In einer bevorzugten Ausführungsform werden zunächst die leichtsiedenden Bestandteile wie beispielsweise Ammoniak und Wasser zuerst abgetrennt und anschließend, falls erforderlich, das Monoalkanolamin von Dialkanolamin oder/und Trialkanolamin bevorzugt destillativ abgetrennt. Die Destillation kann hierbei gemäß sämtlicher geeigneter Verfahren erfolgen.

Die Abtrennung der Mono-, Di- und Triethanolamine kann durch konventionelle Methoden erfolgen. Bevorzugt ist die Destillation, wobei jedoch auch Flüssig/Flüssigextraktion oder Trennungen durch Membranen eingesetzt werden.

Die abgetrennten Bestandteile wie Ammoniak oder Wasser können anschließend in das Verfahren, in dem bevorzugt Monoalkanolamin hergestellt wird, rückgeführt werden. Abgetrenntes Monoalkanolamin kann als Wertprodukt erhalten oder aber ganz oder zum Teil auch einem Verfahren zugeführt werden, in dem bevorzugt Di- oder Trialkanolamin hergestellt wird und in dem das Monoalkanolamin als Edukt dient.

Wird im erfindungsgemäßen Verfahren bevorzugt Dialkanolamin hergestellt, so kann der Produktstrom aus dem Reaktionsraum, der neben dem Dialkanolamin gegebenenfalls Monoalkanolamin oder/und Trialkanolamin sowie Ammoniak und Wasser enthält, einer oder mehreren Trennstufen zugeführt werden, in denen dieses Gemisch aufgetrennt wird. In einer bevorzugten Ausführungsform werden zunächst wiederum die leichtsiedenden Bestandteile wie beispielsweise Ammoniak und Wasser zuerst abgetrennt und anschließend, falls erforderlich, das Dialkanolamin von Monoalkanolamin oder/und Trialkanolamin bevorzugt destillativ abgetrennt. Die Destillation kann hierbei wiederum gemäß sämtlicher geeigneter Verfahren erfolgen.

Die abgetrennten Bestandteile wie Monoalkanolamin, Ammoniak oder Wasser können anschließend in das Verfahren, in dem bevorzugt Dialkanolamin hergestellt wird, rückgeführt werden. Abgetrenntes Dialkanolamin kann als Wertprodukt erhalten oder aber ganz oder zum Teil auch einem Verfahren zugeführt werden, in dem bevorzugt Trialkanolamin hergestellt wird und in dem das Dialkanolamin als Edukt dient.

Wird im erfindungsgemäßen Verfahren bevorzugt Trialkanolamin hergestellt, so kann der Produktstrom aus dem Reaktionsraum, der neben dem Trialkanolamin gegebenenfalls Monoalkanolamin oder/und Dialkanolamin sowie Ammoniak und Wasser enthält, einer oder mehreren Trennstufen zugeführt werden, in denen dieses Gemisch aufgetrennt wird. In einer bevorzugten Ausführungsform werden zunächst wiederum die leichtsiedenden Bestandteile wie beispielsweise Ammoniak und Wasser zuerst abgetrennt und anschließend, falls erforderlich, das Trialkanolamin von Monoalkanolamin oder/und Dialkanolamin bevorzugt destillativ abgetrennt. Die Destillation kann hierbei wiederum gemäß sämtlicher geeigneter Verfahren erfolgen.

Die abgetrennten Bestandteile wie Monoalkanolamin, Dialkanolamin, Ammoniak oder Wasser können anschließend in das Verfahren, in dem bevorzugt Trialkanolamin hergestellt wird, rückgeführt werden. Abgetrenntes Monoalkanolamin kann auch einem weiteren Verfahren zugeführt werden, in dem bevorzugt Dialkanolamin hergestellt wird und in dem das Monoalkanolamin als Edukt dient.

Die Abtrennung der Mono-, Di- und Triethanolamine kann durch konventionelle Methoden erfolgen. Bevorzugt ist die Destillation, wobei jedoch auch Flüssig/Flüssigextraktion oder Trennungen durch Membranen eingesetzt werden.

Ein Vorteil der erfindungsgemäßen Steuerung der Verteilung der verschiedenen Alkanolamine im Produktspektrum über die Regelung des Temperaturprofils im Reaktionsraum ist unter anderem darin zu sehen, daß das Verfahren im Vergleich zu den Verfahren, die im Stand der Technik beschrieben sind, wesentlich flexibler ausgestaltet werden kann. Während im Stand der Technik die Verfahren oder die diesbezüglich beschriebenen Katalysatoren dahingehend optimiert wurden, daß entweder Mono- oder Di- oder Trialkanolamin in bestimmter Ausbeute herstellbar ist, erlaubt das erfindungsgemäße Verfahren über die spezielle Temperaturregelung unter anderem, bevorzugt in Kombination mit der Regelung des Molverhältnisses der Edukte Ammoniak und Alkylenoxid, eine Verfahrensführung, in der in einem einzigen Reaktionsraum in Anwesenheit desselben Katalysators in einer ersten Verfahrensstufe zunächst bevorzugt Mono-oder Di- oder Trialkanolamin hergestellt wird und in einer zweiten Verfahrensstufe über die erfindungsgemäße Regelung bevorzugt ein Alkanolamin hergestellt wird, das von dem in der ersten Verfahrensstufe bevorzugt hergestellten Alkanolamin unterschiedlich ist.

Durch diese flexible Verfahrensführung ist es beispielsweise möglich, variabel auf Kundenwünsche einzugehen oder das Verfahren ohne großen Aufwand an sich ändernde Marktgegebenheiten anzupassen.

Daher beschreibt die vorliegende Erfindung auch ein Verfahren, wie oben beschrieben, das dadurch gekennzeichnet ist, daß
(i) in einer ersten Verfahrensstufe selektiv Monoalkanolamin oder Dialkanolamin oder Trialkanolamin hergestellt wird und
(ii) in einer zweiten Verfahrensstufe im gleichen Reaktionsraum und in Gegenwart des gleichen Katalysators durch Regelung des Temperaturprofils im Reaktionsraum und gegebenenfalls zusätzlich durch das Molverhältnis von Ammoniak und Alkylenoxid die Produktselektivität in der zweiten Verfahrensstufe im Vergleich zur ersten Verfahrensstufe geändert wird.

Selbstverständlich umfaßt diese Ausführungsform des Verfahrens auch Verfahrensführungen, in denen in mindestens einer zusätzlichen Verfahrensstufe ebenfalls im gleichen Reaktionsraum und ebenfalls in Gegenwart des gleichen Katalysators durch Regelung des Temperaturprofils im Reaktionsraum und gegebenenfalls zusätzlich durch das Molverhältnis von Ammoniak zu Alkylenoxid die Produktselektivität in dieser mindestens einen zusätzlichen Verfahrensstufe im Vergleich zur zweiten oder allgemein zur jeweils vorhergehenden Verfahrensstufe geändert wird.

Weitere Möglichkeiten, durch zusätzliche Maßnahmen ebenfalls Einfluß auf die Verteilung der verschiedenen Alkanolamine im Produktspektrum zu nehmen, sind obenstehend beschrieben und können auch beim hier beschriebenen flexiblen Verfahren eingesetzt werden.

Monoalkanolamine werden im Rahmen des erfindungsgemäßen Verfahrens im allgemeinen bei Drücken hergestellt, die im Bereich von 20 bis 250 bar, bevorzugt im Bereich von 40 bis 230 bar und besonders bevorzugt im Bereich von 70 bis 160 bar liegen. Die Temperatur des Ammoniakstromes als auch die Temperatur des Alkylenoxidstromes, die in den Reaktionsraum eingeleitet werden, liegt im allgemeinen im Bereich von 20 bis 200 °C, bevorzugt im Bereich von 50 bis 150 °C und besonders bevorzugt im Bereich von 60 bis 140 °C. Das Molverhältnis von Ammoniak zu Alkylenoxid liegt hierbei im allgemeinen im Bereich von 100 bis 7, bevorzugt im Bereich von 40 bis 7 und besonders bevorzugt im Bereich von 20 bis 7. Die maximale Temperatur im Reaktionsraum liegt weiter im allgemeinen im Bereich von weniger als 200 °C, weiter bevorzugt im Bereich von 20 bis 180 °C, weiter bevorzugt im Bereich von 50 bis 150 °C und besonders bevorzugt im Bereich von 60 bis 130 °C.

Dialkanolamine werden im Rahmen des erfindungsgemäßen Verfahrens im allgemeinen bei Drücken hergestellt, die im Bereich von 20 bis 250 bar, bevorzugt im Bereich von 40 bis 230 bar und besonders bevorzugt im Bereich von 70 bis 160 bar liegen. Die Temperatur des Ammoniakstromes als auch die Temperatur des Alkylenoxidstromes, die in den Reaktionsraum eingeleitet werden, liegt im allgemeinen im Bereich von 20 bis 180 °C, bevorzugt im Bereich von 40 bis 150 °C und besonders bevorzugt im Bereich von 60 bis 140 °C. Das Molverhältnis von Ammoniak zu Alkylenoxid liegt hierbei im allgemeinen im Bereich von 10 bis 2, bevorzugt im Bereich von 8 bis 2 und besonders bevorzugt im Bereich von 7 bis 2. Die maximale Temperatur im Reaktionsraum liegt weiter im allgemeinen im Bereich von 70 bis 200 °C, weiter bevorzugt im Bereich von 75 bis 150 °C und besonders bevorzugt im Bereich von 80 bis 140 °C.

Trialkanolamine werden im Rahmen des erfindungsgemäßen Verfahrens im allgemeinen bei Drücken hergestellt, die im Bereich von 5 bis 250 bar, bevorzugt im Bereich von 30 bis 230 bar und besonders bevorzugt im Bereich von 40 bis 160 bar liegen. Die Temperatur des Ammoniakstromes als auch die Temperatur des Alkylenoxidstromes, die in den Reaktionsraum eingeleitet werden, liegt im allgemeinen im Bereich von 20 bis 180 °C, bevorzugt im Bereich von 40 bis 150 °C und besonders bevorzugt im Bereich von 60 bis 140 °C. Das Molverhältnis von Ammoniak zu Alkylenoxid liegt hierbei im allgemeinen im Bereich von 10 bis 0,3, bevorzugt im Bereich von 8 bis 0,3 und besonders bevorzugt im Bereich von 6 bis 0,3. Die maximale Temperatur im Reaktionsraum liegt weiter im allgemeinen im Bereich von weniger als 400 °C, weiter bevorzugt im Bereich von 75 bis 400 °C, weiter bevorzugt im Bereich von 90 bis 400 °C und besonders bevorzugt im Bereich von 100 bis 400 °C.

Diese Verfahrensführung ist im wesentlichen für sämtliche der oben beschriebenen, als Edukte einsetzbaren Alkylenoxide durchführbar. Bevorzugt wird im Rahmen der vorliegenden Erfindung als Edukt Ethylenoxid eingesetzt.

Daher betrifft die vorliegende Erfindung ein Verfahren, wie oben beschrieben, das dadurch gekennzeichnet ist, daß
(i) in einer ersten Verfahrensstufe selektiv Monoethanolamin hergestellt wird und
(ii) in einer zweiten Verfahrensstufe im gleichen Reaktionsraum und in Gegenwart des gleichen Katalysators durch Regelung des Temperaturprofils im Reaktionsraum und gegebenenfalls zusätzlich durch das Molverhältnis von Ammoniak und Ethylenoxid die Produktselektivität in der zweiten Verfahrensstufe im Vergleich zur ersten Verfahrensstufe geändert wird.

Im Zusammenhang mit dieser bevorzugten Ausführungsform des erfindungsgemäßen Verfahrens, bei der in einer ersten Verfahrensstufe selektiv Monoethanolamin hergestellt wird und in einer zweiten Verfahrensstufe im gleichen Reaktionsraum und in Gegenwart des gleichen Katalysators durch Regelung des Temperaturprofils im Reaktionsraum und gegebenenfalls zusätzlich durch das Molverhältnis von Ammoniak und Ethylenoxid die Produktselektivität in der zweiten Verfahrensstufe im Vergleich zur ersten Verfahrensstufe geändert wird, wird der Begriff "Selektivität" folgendermaßen verwendet:
- das Wertprodukt Monoethanolamin wird dann selektiv hergestellt, wenn im Produktspektrum mehr als 65 Gew.-% Monoethanolamin vorliegen;
- das Wertprodukt Diethanolamin wird dann selektiv hergestellt, wenn im Produktspektrum mehr als 35 Gew.-% Diethanolamin vorliegen;
- das Wertprodukt Triethanolamin wird dann selektiv hergestellt, wenn im Produktspektrum mehr als 35 Gew.-% Triethanolamin vorliegen.

Die Gew.-%-Angaben sind jeweils bezogen auf die Gesamtmenge der hergestellten Ethanolamine.

Demgemäß betrifft die vorliegende Erfindung ein Verfahren, wie oben beschrieben, das dadurch gekennzeichnet ist, daß
(a) in (i) mehr als 65 Gew.-% Monoethanolamin und in (ii) mehr als 35 Gew.- % Di- oder Triethanolamin,
jeweils bezogen auf die Gesamtmenge aus Mono-, Di- und Triethanolamin, gebildet werden.

Bezüglich der erfindungsgemäßen Verfahrensführung ist zu bemerken, daß der Druck mindestens so gewählt wird, daß das fluide Reaktionsgemisch einphasig ohne Vorhandensein einer Gasphase vorliegt. Der Mindestdruck ergibt sich dabei aufgrund der Thermodynamik als Dampfdruck des Reaktionsgemischs bei der entsprechenden Temperatur, d.h. wird durch die Konzentration der Komponenten und der Temperatur im einzelnen Reaktorabschnitt bestimmt.

Dabei ist es vorteilhaft, aber nicht notwendig, daß der Reaktor in seinem gesamten Volumen bei einem einzigen Druck betrieben wird. Andernfalls sind beispielsweise Abschnitte mit fallendem Systemdruck möglich und leicht zu realisieren, wobei beispielsweise der Mindestdruck z.B. durch die Konzentration der leichtsiedensten Komponenten im Reaktionsgemsich bei der Temperatur des Hotspots durch die Thermodynamik bestimmt wird.

Wird im erfindungsgemäßen Verfahren beispielsweise in einer ersten Verfahrensstufe Monoethanolamin hergestellt, so wird im allgemeinen in Druckbereichen von 20 bis 250 bar, vorzugsweise von 40 bis 230 bar und besonders bevorzugt von 70 bis 160 bar gearbeitet. Die Verweilzeiten des Reaktionsgutes im Reaktor liegen im allgemeinen im Bereich von 2 bis 60 Minuten, vorzugsweise im Bereich von 5 bis 20 Minuten. Das Molverhältnis von Ammoniak und Ethylenoxid liegt im allgemeinen im Bereich von 5 bis 100, bevorzugt im Bereich von 5 bis 40 und weiter bevorzugt im Bereich von 5 bis 20 Mol. Hierbei wird bei Temperaturgradienten, d.h. bei Temperaturdifferenzen zwischen Maximaltemperatur im Reaktionsraum und Temperatur der nichtvermischten Edukte gearbeitet, die im Bereich von im allgemeinen 0 bis 100 °C, bevorzugt von 0 bis 80 °C und weiter bevorzugt von 0 bis 30 °C liegen.

Soll in der zweiten Verfahrensstufe im gleichen Reaktionsraum in Anwesenheit desselben Katalysators selektiv Diethanolamin hergestellt werden, so wird bei den Druckbereichen und Verweilzeitbereichen, die bei der Herstellung des Monoethanolamins gewählt werden, bei Temperaturgradienten gearbeitet, die im Bereich von im allgemeinen 50 bis 120 °C, bevorzugt von 60 bis 100 °C liegen.

Soll in der zweiten Verfahrensstufe im gleichen Reaktionsraum in Anwesenheit desselben Katalysators selektiv Triethanolamin hergestellt werden, so wird bei den Druckbereichen und Verweilzeitbereichen, die bei der Herstellung des Monoethanolamins oder/und des Diethanolamins gewählt werden, bei Temperaturgradienten gearbeitet, die im Bereich von im allgemeinen 70 bis 300 °C.

Die hier beschriebenen Umsetzungen bei den angegebenen Reaktionsparametern beziehen sich auf die erfindungsgemäß vorzugsweise verwendeten nicht rückvermischenden Rohrreaktoren, sind jedoch auch auf ebenfalls verwendbare Rührkessel, Anordnungen von Rührkessel zu Rührkesselkaskaden anwendbar bzw. übertragbar.

In den folgenden Beispielen wird die Erfindung näher erläutert.

### Beispiele

### Beispiel 1: Herstellung des Katalysators 1

400 g Pentasilzeolith der Struktur ZBM-10, hergestellt gemäß der DE-A 43 23 774.6 wurde mit dem Alexanderwerk der Siebweite 1 mm gemahlen.

Anschließend wurde der gemahlene Katalysator zusammen mit 100 g Plural® der Fa. Condea und 10 g Ameisensäure geknetet, wobei 400 ml Wasser zugesetzt wurden. Nach einer Knetzeit von 60 min wurden nochmals 100 ml Wasser zugesetzt.

Nach einer Knetzeit von insgesamt 75 min wurde die Knetmasse in einer Stranpresse bei einem Druck von 50 bar in 3 mm-Stränge verarbeitet.

Anschließend wurde das erhaltene Material 16 Stunden bei 120 °C getrocknet und 5 Stunden bei 500 °C unter Luft calciniert.

### Beispiel 2: Herstellung des Katalysators 2

In 1000 ml wasserfreiem Ethanol, der über einem 3 Ångstrøm Molsieb vorgetrocknet wurde, wurden 16 g 3-Aminopropyltriethoxysilan gelöst und 100 g des Katalysators gemäß Beispiel 1 in einem Kolben hinzugefügt.

Für die Dauer von 10 Stunden wurde der Ansatz am Rotationsverdampfer bei geringer Drehzahl durchmischt. Anschließend wurde das Lösungsmittel abgedampft.

Das erhaltene Material wurde mit einer Heizrate von 2 °C/min auf eine Temperatur von 550 °C aufgeheizt und 3 Stunden bei 550 °C unter Luft calciniert.

### Beispiel 3: Herstellung des Katalysators 3

Dieses Beispiel entspricht dem Referenzbeispiel 3 der EP-A 0 941 986, auf die im Kontext der vorliegenden Anmeldung Bezug genommen wird.

### Beispiel 4: Herstellung des Katalysators 4 (La/Montmorillonit)

Dieses Beispiel entspricht dem Katalysator E der EP-A 0 652 207, auf die im Kontext der vorliegenden Anmeldung Bezug genommen wird.

### Beispiele 5 bis 7: Herstellung von Ethanolaminen

Die Feedtemperatur des Ammoniakstroms betrug 70 °C. Die Temperatur des Ethylenoxidstroms war 25 °C. Der Temperaturgradient wurde so gewählt, daß im ersten Reaktorabschnitt von 5 % der Reaktionsstrecke (identisch 5 % des Reaktionsvolumens) der steilste Temperaturanstieg auftrat. Es wurden die Temperaturen im nachfolgenden Rohrreaktorsystem aus 2 bis 7 unabhängig temperierten Rohrabschnitten konstant gehalten bei gewollten, im einzelnen Versuch einheitlichen Temperaturen zwischen 90 und 160 °C.

Die Temperaturregelung erfolgte wie oben beschrieben durch Anpassung der Eintrittstemperatur des einzelnen Kühlmittelstroms. Die Temperatur wurde in der Mischstelle und mit je zwei Thermoelementen in jedem Rohrabschnitt im Inneren des Reaktionsrohres gemessen sowie zusätzlich die Ein- und Austrittstemperaturen des Kühlmediums in die Doppelmäntel der Rohrabschnitte.

Durch einen Rohrreaktor des Innendurchmessers 4 mm und einer Länge von 3 m wurden bei einem Druck im Bereich von 110 bis 120 bar Ethylenoxid und Ammoniak bei einer Verweilzeit von ungefähr 3,3 min geleitet. Die Verweilzeiten wurden berechnet auf der Basis der Reinstoffdichten von Ethylenoxid und Ammoniak unter Reaktionsbedingungen und sind bezogen auf das Volumen des Leerrohres.

Der Rohrreaktor war hierbei mit jeweils 15 g der in Tabelle 1 angegebenen Katalysatoren gefüllt.

Die Temperatur des Reaktors wurde mittels eines temperierten Ölkreislaufs durch einen Doppelmantel um das Reaktionsrohr eingestellt.

Der Reaktionsaustrag wurde gaschromatographisch analysiert, wobei die in Tabelle 1 angegebenen Zusammensetzungen ermittelt wurden.

**Tabelle 1**

| Beispiel | Katalysator | Molverhältnis NH₃/EO | Temp./ °C | Selektivitäten / Gew.-% | | |
|---|---|---|---|---|---|---|
| | | | | MEA | DEA | TEA |
| 5 | 1 | 10 | 90 | 71 | 28 | 1 |
| 6 | 1 | 4 | 90 | 42 | 40 | 17 |
| 7 | 1 | 10 | 160 | 26 | 28 | 46 |

Die Abkürzungen EO, MEA, DEA und TEA stehen dabei für Ethylenoxid, Monoethanolamin, Diethanolamin und Triethanolamin. Die Selektivität ist dabei definiert als Gew.-% Alkanolamin / Gew.-% der in der Summe gebildeten Ethanolamine.

### Beispiel 8: Flexible Herstellung von Ethanolaminen

Dieses Beispiel wurde analog Beispiel 5 bis 7 durchgeführt, wobei anstelle eines Katalysators ein Gemisch aus Wasser und 15% Ammoniak eingesetzt wurde.

**Tabelle 2**

| Beispiel | Katalysator | Molverhältnis NH₃/EO | Temp./ °C | Selektivitäten / Gew.-% | | |
|---|---|---|---|---|---|---|
| | | | | MEA | DEA | TEA |
| 11 | Wasser, 15 % | 6 | 90 | 70 | 23 | 7 |
| 12 | Wasser, 15 % | 6 | 130 | 38 | 37 | 25 |
| 13 | Wasser, 15 % | 6 | 170 | 17 | 28 | 55 |

### Beispiele 9 bis 13: Selektive Herstellung von Diethanolamin

### (a) Selektive Herstellung von Monoethanolamin

Durch einen Rohrreaktor des Innendurchmessers 4 mm und einer Länge von 3 m wurden bei einem Druck im Bereich von 110 bis 120 bar Ethylenoxid und Ammoniak bei einer Verweilzeit von ungefähr 3,3 min geleitet. Die Verweilzeiten wurden berechnet auf der Basis der Reinstoffdichten von Ethylenoxid und Ammoniak unter Reaktionsbedingungen und sind bezogen auf das Volumen des Leerrohres.

Der Rohrreaktor war hierbei mit jeweils 15 g der in Tabelle 2 angegebenen Katalysatoren gefüllt.
Die Temperatur des Reaktors wurde mittels eines temperierten Ölkreislaufs durch einen Doppelmantel um das Reaktionsrohr eingestellt, wobei die Feedtemperatur 70 °C betrug.

Der Reaktionsaustrag wurde gaschromatographisch analysiert, wobei die in Tabelle 3 angegebenen Zusammensetzungen ermittelt wurden.

**Tabelle 3**

| Beispiel | Katalysator | Molverhältnis NH₃/EO | Temp./ °C | Selektivitäten / % | | |
|---|---|---|---|---|---|---|
| | | | | MEA | DEA | TEA |
| 9 | Dowex® 50X8 | 60 | 100 | 92 | 8 | 0 |
| 10 | 4 | 25 | 95 | 90 | 10 | 0 |
| V1 | 15 Gew.-% Wasser | 10 | 100 | 60 | 30 | 10 |

Dem Ammoniakstrom wurde Wasser derart zugesetzt, daß sich die Wasserkonzentration von 15 Gew.-% im wasserhaltigen Ammoniakstrom ergab. In den weiteren Beispielen, in denen nicht ausdrücklich auf die Gegenwart von Wasser im Feed verwiesen wurde, wurde wasserfreier Ammoniak eingesetzt.

Die Abkürzungen EO, MEA, DEA und TEA stehen dabei für Ethylenoxid, Monoethanolamin, Diethanolamin und Triethanolamin. Die Selektivität ist dabei definiert als das Verhältnis der Gew.-% der Alkanolamine / zur Summe der Gew.- % der gebildeten Ethanolamine.

### (b) Selektive Herstellung von Diethanolamin

Monoethanolamin wurde im Rohrreaktor mit Ethylenoxid am Festbett umgesetzt. Die Reaktionsführung wurde wie in den Beispielen 9 bis 13 (a) vorgenommen.

Der Reaktionsaustrag wurde gaschromatographisch analysiert, wobei die in Tabelle 4 angegebenen Zusammensetzungen ermittelt wurden.

**Tabelle 4**

| Beispiel | Katalysator | Molverhältnis MEA/EO | Temp./°C | Selektivitäten / % | |
|---|---|---|---|---|---|
| | | | | DEA | TEA |
| 11 | 3 | 4 | 110 | 90 | 10 |
| V2 | Autokatalyse | 4 | 110 | 86 | 14 |

Das mit V2 bezeichnete Vergleichsbeispiel wurde ohne Zusatz eines Katalysators durchgeführt.

### (c) Kombination der Verfahren zur selektiven MEA-Synthese gemäß (a) mit dem Verfahren zur selektiven Herstellung von Diethanolamin gemäß (b)

In Tabelle 5 sind die Ergebnisse von Versuchen angegeben, die in einem Kombinationsverfahren unter Verwendung von zwei Reaktoren erzielt wurden.

**Tabelle 5**

| Beispiel | Katalysator I | Katalysator II | Temp./ °C Reaktor 1/2 | Selektivitäten | |
|---|---|---|---|---|---|
| | | | | DEA | TEA |
| 12 | Dowex® 50X8 | 3 | 100/110 | 91 | 9 |
| 13 | 4 | 3 | 95/110 | 91 | 9 |
| V3 | Dowex® 50X8 | Autokatalyse | 110/110 | 87 | 13 |

## Patentansprüche

1. Verfahren zur Herstellung von Ethanolaminen durch Umsetzung von Ammoniak mit Ethylenoxid in Gegenwart eines Katalysators in einem Reaktionsraum, bei dem
(i) in einer ersten Verfahrensstufe Monoethanolamin mit einer Selektivität von > 65 Gew.-%, bezogen auf die Gesamtmenge aus Mono-, Di- und Triethanolamin, hergestellt wird, und
(ii) in einer weiteren Verfahrensstufe Diethanolamin mit einer Selektivität von > 35% oder Triethanolamin mit einer Selektivität von > 35 Gew.-%, bezogen auf die Gesamtmenge aus Mono-, Di- und Triethanolamin, hergestellt wird,
wobei die Verfahrensstufen (i) und (ii) im gleichen Reaktionsraum und in Gegenwart des gleichen Katalysators bei einem Druck von 20 bis 250 bar und einer Verweilzeit von 2 bis 60 Minuten durchgeführt werden,
wobei
die erste Verfahrensstufe (i) bei einer Maximaltemperatur im Reaktionsraum von 20 bis 180 °C, wobei die Temperaturdifferenz zwischen Maximaltemperatur und Temperatur der nicht vermischten Edukte 0 bis 100 °C beträgt, durchgeführt wird, und
die weitere Verfahrensstufe (ii), sofern Diethanolamin mit einer Selektivität von > 35 Gew.-% hergestellt wird, bei einer Maximaltemperatur von 70 bis 200 °C, wobei die Temperaturdifferenz zwischen Maximaltemperatur und Temperatur der nicht vermischten Edukte 50 bis 120 °C beträgt, durchgeführt wird
oder, sofern Triethanolamin mit einer Selektivität von > 35 Gew.-% hergestellt wird, bei einer Maximaltemperatur im Reaktionsraum von 75 bis 400 °C, wobei die Temperaturdifferenz zwischen Maximaltemperatur und Temperatur der nicht vermischten Edukte 70 bis 300 °C beträgt, durchgeführt wird,
wobei die Verfahrensstufe (ii) auch vor der Verfahrensstufe (i) durchgeführt werden kann.

2. Verfahren nach Anspruch 1, **dadurch gekennzeichnet, dass** das Molverhältnis Ammoniak zu Ethylenoxid im Bereich von 5 bis 100 liegt.

3. Verfahren nach Anspruch 1 oder 2, **dadurch gekennzeichnet, dass** die Temperaturdifferenz zwischen Maximaltemperatur und Temperatur der nicht vermischten Edukte in der ersten Verfahrensstufe (i) 0 bis 30 °C beträgt.

4. Verfahren nach einem der Ansprüche 1 bis 3, **dadurch gekennzeichnet, dass** die Temperaturdifferenz zwischen Maximaltemperatur und Temperatur der nicht vermischten Edukte in der weiteren Verfahrensstufe (ii), sofern Diethanolamin mit einer Selektivität von > 35 Gew.-% hergestellt wird, 60 bis 100 °C beträgt.

5. Verfahren nach einem der Ansprüche 1 bis 4, **dadurch gekennzeichnet, dass** der Katalysator ein Zeolithkatalysator ist.

6. Verfahren nach einem der Ansprüche 1 bis 4, **dadurch gekennzeichnet, dass** der Katalysator Wasser ist.

## Claims

1. A process for preparing ethanolamines by reaction of ammonia with ethylene oxide in the presence of a catalyst in a reaction space, which process comprises
(i) a first step of preparing monoethanolamine with a selectivity of >65% by weight, based on the total amount of mono-, di- and triethanolamine, and
(ii) a further step of preparing diethanolamine with a selectivity of >35% or triethanolamine with a selectivity of >35% by weight, based on the total amount of mono-, di- and triethanolamine,
wherein said steps (i) and (ii) are carried out in the same reaction space and in the presence of the same catalyst at a pressure of 20 to 250 bar with a residence time of 2 to 60 minutes,
wherein
said first step (i) is carried out at a maximum temperature in the reaction space of 20 to 180°C, wherein the temperature difference between the maximum temperature and the temperature of the unmixed reactants is in the range from 0 to 100°C,
and
said further step (ii) is carried out at a maximum temperature of 70 to 200°C when diethanolamine is prepared with a selectivity of >35% by weight, wherein the temperature difference between the maximum temperature and the temperature of the unmixed reactants is in the range from 50 to 120°C or
at a maximum temperature in the reaction space of 75 to 400°C when triethanolamine is prepared with a selectivity of >35% by weight, wherein the temperature difference between the maximum temperature and the temperature of the unmixed reactants is in the range from 70 to 300°C,
wherein said step (ii) can also be carried out before said step (i).

2. The process according to claim 1 wherein the molar ratio of ammonia to ethylene oxide is in the range from 5 to 100.

3. The process according to claim 1 or 2 wherein the temperature difference between the maximum temperature and the temperature of the unmixed reactants is in the range from 0 to 30°C in said first step (i).

4. The process according to any of claims 1 to 3 wherein the temperature difference between the maximum temperature and the temperature of the unmixed reactants is in the range from 60 to 100°C in said further step (ii) when diethanolamine is prepared with a selectivity of >35% by weight.

5. The process according to any of claims 1 to 4 wherein the catalyst is a zeolite catalyst.

6. The process according to any of claims 1 to 4 wherein the catalyst is water.

## Revendications

1. Procédé pour la préparation d'éthanolamines par transformation d'ammoniac avec de l'oxyde d'éthylène en présence d'un catalyseur dans un espace de réaction, dans lequel
(i) on prépare, dans une première étape de procédé, de la monoéthanolamine à une sélectivité > 65% en poids, par rapport à la quantité totale de monoéthanolamine, de diéthanolamine et de triéthanolamine, et
(ii) on prépare, dans une autre étape de procédé, de la diéthanolamine à une sélectivité > 35% ou de la triéthanolamine à une sélectivité > 35% en poids, par rapport à la quantité totale de monoéthanolamine, de diéthanolamine et de triéthanolamine,
les étapes de procédé (i) et (ii) étant réalisées dans le même espace de réaction et en présence du même catalyseur à une pression de 20 à 250 bars et pendant un temps de séjour de 2 à 60 minutes,
la première étape de procédé (i) étant réalisée à une température maximale dans l'espace de réaction de 20 à 180°C, la différence de température entre la température maximale et la température des produits de départ non mélangés valant 0 à 100°C, et
l'autre étape de procédé (ii), pour autant que la diéthanolamine soit préparée à une sélectivité > 35% en poids, étant réalisée à une température maximale de 70 à 200°C, la différence de température entre la température maximale et la température des produits de départ non mélangés valant 50 à 120°C ou,
pour autant que la triéthanolamine soit préparée à une sélectivité > 35% en poids, étant réalisées à une température maximale dans l'espace de réaction de 75 à 400°C, la différence de température entre la température maximale et la température des produits de départ non mélangés valant 70 à 300°C,
l'étape de procédé (ii) pouvant également être réalisée avant l'étape de procédé (i).

2. Procédé selon la revendication 1, **caractérisé en ce que** le rapport molaire ammoniac à oxyde d'éthylène se situe dans la plage de 5 à 100.

3. Procédé selon la revendication 1 ou 2, **caractérisé en ce que** la différence de température entre la température maximale et la température des produits de départ non mélangés dans la première étape de procédé (i) vaut 0 à 30°C.

4. Procédé selon l'une quelconque des revendications 1 à 3, **caractérisé en ce que** la différence de température entre la température maximale et la température des produits de départ non mélangés dans l'autre étape de procédé (ii), pour autant la diéthanolamine soit préparée à une sélectivité > 35% en poids, vaut 60 à 100°C.

5. Procédé selon l'une quelconque des revendications 1 à 4, **caractérisé en ce que** le catalyseur est un catalyseur zéolithique.

6. Procédé selon l'une quelconque des revendications 1 à 4, **caractérisé en ce que** le catalyseur est l'eau.
